# EUROPEAN PATENT APPLICATION

(11) **EP 4 083 055 A1**
(43) Date of publication of application: **02.11.2022**
(21) Application number: 20907310.5
(22) Date of filing: 15.12.2020
(51) Int. Cl.: C07K 14/415, C12C 5/00

(54) **SUGAR-MODIFIED PROTEIN**

(30) Priority: 27.12.2019 JP 2019239831
(71) Applicant: Suntory Holdings Limited, Osaka 530-8203 (JP)
(72) Inventor: IWASA, Keiko, Soraku-gun, Kyoto 619-0284 (JP); BEPPU, Yoshinori, Soraku-gun, Kyoto 619-0284 (JP); NAKAHARA, Koichi, Soraku-gun, Kyoto 619-0284 (JP); MATSUO, Yoshihide, Soraku-gun, Kyoto 619-0284 (JP); FUJITA, Yohei, Mishima-gun, Osaka 618-0001 (JP)
(74) Representative: Vossius & Partner Patentanwälte Rechtsanwälte mbB
(86) International application number: PCT/JP2020/046833
(87) International publication number: WO 2021/131923

(57) **Abstract**

The present invention aims to provide a substance useful for imparting a rich flavor to a food or beverage, and a method of imparting a rich flavor to a food or beverage. The present invention relates to a glycated protein having a glycated side chain amino group of lysine, the glycated protein having a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
(a1) a protein having the amino acid sequence represented by SEQ ID NO: 1;
(a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
(a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine.

## Description

### TECHNICAL FIELD

The present invention relates to a glycated protein. More specifically, the present invention relates to a glycated protein having a glycated side chain amino group of lysine at a specific position in the protein. The present invention also relates to a rich flavor imparting agent. The present invention still also relates to a method of imparting a rich flavor to a food or beverage.

### BACKGROUND ART

Diversification of consumer preferences in recent years has created a desire for development of beer-taste alcoholic beverages, non-alcoholic beer-taste beverages, and the like with various aromatic characteristics.

For example, Patent Literature 1 discloses use of an alcohol component, a sweet component, an aldehyde-based component, or the like as a flavor improver for beer-like beverages in order to enhance the rich taste or the like of beer-like beverages.

### CITATION LIST

### - Patent Literature

Patent Literature 1: JP 2016-214262 A

### SUMMARY OF INVENTION

### - Technical Problem

The present invention aims to provide a substance useful for imparting a rich flavor to a food or beverage. The present invention also aims to provide a method of imparting a rich flavor to a food or beverage.

### - Solution to Problem

As a result of studies on substances capable of imparting a rich flavor to food or beverages, the present inventors found that, for example, a glycated protein having an amino acid sequence represented by SEQ ID NO: 1 and having a glycated side chain amino group of at least one of lysine at position 160 or lysine at position 189 of the amino acid sequence is a substance that correlates with a rich flavor. The present inventors also found that a protein having an amino acid sequence represented by SEQ ID NO: 2 and having a glycated side chain amino group of lysine at a position corresponding to position 117 of the amino acid sequence is also a substance that correlates with a rich flavor.

Specifically, the present invention relates to a glycated protein, a rich flavor imparting agent, a method of imparting a rich flavor to a food or beverage, and the like described below, but the present invention is not limited thereto.
(1) A glycated protein having a glycated side chain amino group of lysine, the glycated protein having a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
   (a1) a protein having the amino acid sequence represented by SEQ ID NO: 1;
   (a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
   (a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine.
(2) The glycated protein according to (1) above, wherein the protein according to any one of (a1) to (a3) is a barley-derived protein.
(3) A glycated protein having a glycated side chain amino group of lysine, the glycated protein having a glycated side chain amino group of lysine at a position corresponding to position 117 of an amino acid sequence represented by SEQ ID NO: 2 of a protein according to any one of (b1) to (b3) below:
   (b1) a protein having the amino acid sequence represented by SEQ ID NO: 2;
   (b2) a protein having an amino acid sequence wherein 1 to 9 amino acids are deleted, substituted, inserted, and/or added in a region other than lysine at position 117 of the amino acid sequence represented by SEQ ID NO: 2; and
   (b3) a protein having an amino acid sequence having at least 98% identity with the amino acid sequence represented by SEQ ID NO: 2, in which an amino acid at a position corresponding to position 117 of the amino acid sequence represented by SEQ ID NO: 2 is lysine.
(4) The glycated protein according to (3) above, wherein the protein according to any one of (b1) to (b3) is a barley-derived protein.
(5) A rich flavor imparting agent containing at least one of a glycated protein (A) or a glycated protein (B) below:
   glycated protein (A): a glycated protein having a glycated side chain amino group of lysine, the glycated protein having a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
      (a1) a protein having the amino acid sequence represented by SEQ ID NO: 1;
      (a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
      (a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine;
   glycated protein (B): a glycated protein having a glycated side chain amino group of lysine, the glycated protein having a glycated side chain amino group of lysine at a position corresponding to position 117 of an amino acid sequence represented by SEQ ID NO: 2 of a protein according to any one of (b1) to (b3) below:
      (b1) a protein having the amino acid sequence represented by SEQ ID NO: 2;
      (b2) a protein having an amino acid sequence wherein 1 to 9 amino acids are deleted, substituted, inserted, and/or added in a region other than lysine at position 117 of the amino acid sequence represented by SEQ ID NO: 2; and
      (b3) a protein having an amino acid sequence having at least 98% identity with the amino acid sequence represented by SEQ ID NO: 2, in which an amino acid at a position corresponding to position 117 of the amino acid sequence represented by SEQ ID NO: 2 is lysine.
(6) The rich flavor imparting agent according to (5) above for use in imparting a rich flavor to a beer-taste alcoholic beverage or a non-alcoholic beer-taste beverage.
(7) Use of at least one of the glycated protein (A) or the glycated protein (B) for imparting a rich flavor to a food or beverage.
(8) The use according to (7) above, wherein the food or beverage is a beer-taste alcoholic beverage or a non-alcoholic beer-taste beverage.
(9) A method of imparting a rich flavor to a food or beverage, the method including adding at least one of the glycated protein (A) or the glycated protein (B) to a food or beverage.
(10) The method according to (9) above, wherein the food or beverage is a beer-taste alcoholic beverage or a non-alcoholic beer-taste beverage.

### - Advantageous Effects of Invention

The present invention can provide a glycated protein useful for imparting a rich flavor to a food or beverage. The present invention can also provide a method of imparting a rich flavor to a food or beverage.

### BRIEF DESCRIPTION OF DRAWINGS

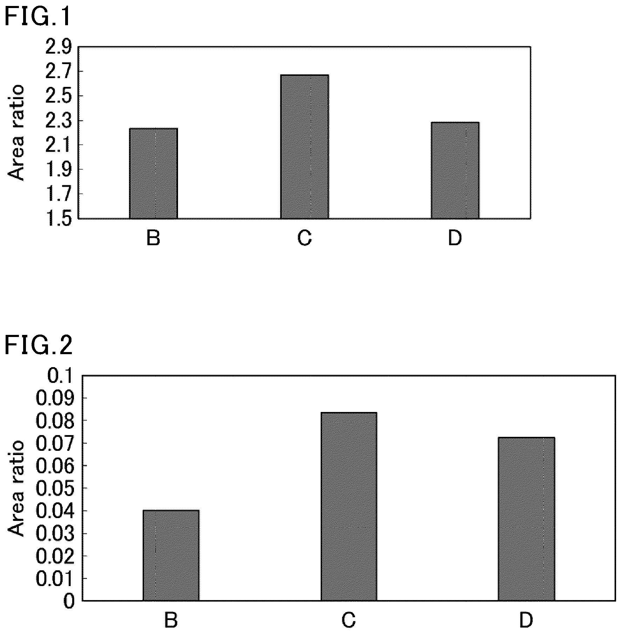

FIG. 1 is a graph showing a ratio of ((sum of areas of glycated Z4 (142-174) peptide ions)/(area of unmodified Z4 (142-174) peptide ions)) in each of glycated proteins purified from three different beers (B, C, and D). Specifically, the "(area of unmodified Z4 (142-174) peptide ions)" is the area of ions obtained by LC-MS from an unmodified peptide fragment (unmodified Z4 (142-174) peptide) consisting of amino acids at positions to 142 to 174 of an amino acid sequence of SerpinZ4 (SEQ ID NO: 1); and the "(sum of areas of glycated Z4 (142-174) peptide ions)" is the sum of the areas of ions obtained by LC-MS from a glycated Z4 (142-174) peptide having one hexose or two hexoses (dihexose) bound to K160 of the peptide.
FIG. 2 is a graph showing a ratio of ((sum of areas of glycated Z4 (182-200) peptide ions)/(area of unmodified Z4 (182-200) peptide ions)) in each of glycated proteins purified from three different beers (B, C, and D). Specifically, the "(area of unmodified Z4 (182-200) peptide ions)" is the area of ions obtained by LC-MS from an unmodified peptide fragment (unmodified Z4 (182-200) peptide) consisting of amino acids at positions 182 to 200 of an amino acid sequence of SerpinZ4 (SEQ ID NO: 1); and the "(sum of areas of glycated Z4 (182-200) peptide ions)" is the sum of the areas of ions obtained by LC-MS from a glycated Z4 (182-200) peptide having one hexose or two hexoses (dihexose) bound to lysine at position 189 (K189) of the peptide.

### DESCRIPTION OF EMBODIMENTS

In a first embodiment, the present invention provides a glycated protein having a glycated side chain amino group of lysine, the glycated protein having a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
(a1) a protein having the amino acid sequence represented by SEQ ID NO: 1;
(a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
(a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine.

The glycated protein is also referred to as "the glycated protein according to the first embodiment of the present invention".

In the amino acid sequence of the glycated protein according to the first embodiment of the present invention, at least one of an amino acid at a position corresponding to position 160 or an amino acid at a position corresponding to position 189 of the amino acid sequence represented by SEQ ID NO: 1 is lysine. The glycated protein according to the first embodiment of the present invention is a glycated protein having a glycated side chain amino group of at least one of lysine at a position corresponding to position 160 or lysine at a position corresponding to position 189 of the amino acid sequence represented by SEQ ID NO: 1.

Herein, the phrase "position corresponding to position 160 of the amino acid sequence represented by SEQ ID NO: 1" means a position corresponding to position 160 (160th position) of the amino acid sequence of SEQ ID NO: 1 (reference amino acid sequence) when an amino acid sequence (primary structure) of a protein (polypeptide) as a comparison target is aligned based on amino acid sequence homology with the amino acid sequence of SEQ ID NO: 1. Thus, the amino acid at a position corresponding to position 160 of the amino acid sequence represented by SEQ ID NO: 1 is lysine at position 160 of SEQ ID NO: 1, but may not be at position 160 in a polypeptide having an amino acid sequence different from the amino acid sequence represented by SEQ ID NO: 1. The same applies to an amino acid other than the amino acid at position 160 of the acid sequence represented by SEQ ID NO: 1, for example, lysine at a position corresponding to lysine at position 189 of the amino acid sequence represented by SEQ ID NO: 1. The same applies to lysine at a position corresponding lysine at position 117 of an amino acid sequence represented by SEQ ID NO: 2 (described later). Herein, the amino acid number is counted from the N-terminal side. Herein, the position in the amino acid sequence which corresponds to lysine at position 160 of the amino acid sequence represented by SEQ ID NO: 1 is also referred to as a "position corresponding to position 160 of SEQ ID NO: 1". Likewise, the position in the amino acid sequence which corresponds to lysine at position 189 of the amino acid sequence represented by SEQ ID NO: 1 is also referred to as a "position corresponding to position 189 of SEQ ID NO: 1".

Lysine at a position corresponding to position 160 and/or lysine at a position corresponding to position 189 of SEQ ID NO: 1 can be easily identified in any of the proteins (polypeptides) (a1), (a2), and (a3) above.

Lysine at a position corresponding to position 160 and/or lysine at a position corresponding to position 189 of SEQ ID NO: 1 can be identified, for example, using a known sequence comparison program such as ClustalW or Protein Blast of NCBI. Usually, default parameters can be used in ClustalW. ClustalW is available at the websites of Data Bank of Japan (DDBJ), European Bioinformatics Institute (EBI), and the like. Amino acid sequences of two polypeptides to be compared are input into the system, and the program is executed, whereby alignment based on amino acid sequence homology can be performed. Here, the amino acid sequence of SEQ ID NO: 1 is input as one of the two polypeptides, which makes it possible to easily identify a position in the polypeptide of SEQ ID NO: 1 to which a residue at a specific position of the other polypeptide corresponds.

For example, in the amino acid sequence represented by SEQ ID NO: 2, the amino acid at a position corresponding to position 189 of SEQ ID NO: 1 is lysine at position 190.

In the present invention, the phrase "having a glycated side chain amino group of lysine" means that a sugar is bound to a side chain amino group of lysine.

The sugar may be a monosaccharide, a disaccharide, or a trisaccharide or higher oligosaccharide or polysaccharide. The sugar is preferably a monosaccharide, disaccharide, or trisaccharide, more preferably a monosaccharide or disaccharide.

The monosaccharide may be a reducing sugar. Examples thereof include all monosaccharides including glucose, fructose, galactose, mannose, rhamnose, arabinose, and xylose. The monosaccharide is preferably D-form.

The disaccharide may be a reducing sugar. Examples thereof include maltose type disaccharides such as maltose, isomaltose, and lactose.

Examples of the trisaccharide include maltotriose, isomaltotriose, cellotriose, fucosyllactose, and sialyllactose.

In one embodiment, the sugar is preferably a sugar contained in beer. In one embodiment, the monosaccharide bound to a side chain amino group of lysine is preferably glucose, arabinose, xylose, or the like, for example. The disaccharide is preferably maltose, isomaltose, or the like. The trisaccharide is preferably maltotriose, isomaltotriose, or the like.

The amino acid sequence represented by SEQ ID NO: 1 is an amino acid sequence of SerpinZ4 (aliases: BSZ4, HorvuZ4, Major endosperm albumin, SerpinZ4, Serpin-Z4, Protein Z, and Protein Z4) derived from barley (binomial name: *Hordeum vulgare*).

In the protein according to (a1) above, the position corresponding to position 160 of SEQ ID NO: 1 is position 160 of the amino acid sequence of SerpinZ4, and the position corresponding to 189 is position 189 of the amino acid sequence thereof.

Herein, the phrase "1 to 9 amino acids are deleted, substituted, inserted, and/or added in the amino acid sequence of the protein" means that 1 to 9 amino acids are deleted, substituted, inserted, and/or added at any 1 to 9 positions in the same amino acid sequence. Two or more of the deletion, replacement, insertion, and addition may be caused simultaneously.

The number of amino acids deleted, substituted, inserted, and/or added in the amino acid sequence of the protein according to (a2) above is preferably 1 to 8, 1 to 7, or 1 to 6, more preferably 1 to 5, still more preferably 1 to 4, particularly preferably 1 to 3, most preferably 1 or 2.

In the protein of (a3) above, preferably, the amino acid sequence has at least 99% identity (sequence identity) with the amino acid sequence represented by SEQ ID NO: 1.

The identity of the amino acid sequence can be calculated with default parameters using analysis software such as BLAST, for example.

In one embodiment, the glycated protein according to the first embodiment of the present invention is preferably a glycated protein having a glycated side chain amino group of at least one of lysine at a position corresponding to position 160 or lysine at a position corresponding to position 189 of the amino acid sequence represented by SEQ ID NO: 1 of the protein according to (a1) above (protein having the amino acid sequence represented by SEQ ID NO: 1). The glycated protein can also be referred to as a glycated protein having the amino acid sequence represented by SEQ ID NO: 1 and having a glycated side chain amino group of at least one of lysine at position 160 or lysine at position 189 of the amino acid sequence.

In one embodiment, the glycated protein according to the first embodiment of the present invention is preferably a glycated protein having a glycated side chain amino group of lysine at a position corresponding to position 160 of the amino acid sequence represented by SEQ ID NO: 1 of the protein according to any one of (a1) to (a3) above (preferably, the protein (a1)).

The glycated protein of the present invention may have a glycated side chain amino group of lysine other than at least one of lysine at a position corresponding to position 160 or lysine at a position corresponding to position 189 of the amino acid sequence represented by SEQ ID NO: 1 of any the proteins of (a1), (a2), and (a3) above.

The production process and source of the glycated protein according to the first embodiment of the present invention are not limited. In one embodiment, the glycated protein according to the first embodiment of the present invention is preferably a grain-derived protein having a glycated side chain amino group of at least one of lysine at a position corresponding to position 160 or lysine at a position corresponding to position 189 of the amino acid sequence of SEQ ID NO: 1. Examples of grains include barley, wheat, corn, and rice. Of these, barley is preferred. Preferably, the proteins (a1) to (a3) are barley-derived proteins.

In a second embodiment, the present invention provides a glycated protein having a glycated side chain amino group of lysine, the glycated protein having a glycated side chain amino group of lysine at a position corresponding to position 117 of an amino acid sequence represented by SEQ ID NO: 2 of a protein according to any one of (b1) to (b3) below:
(b1) a protein having the amino acid sequence represented by SEQ ID NO: 2;
(b2) a protein having an amino acid sequence wherein 1 to 9 amino acids are deleted, substituted, inserted, and/or added in a region other than lysine at position 117 of the amino acid sequence represented by SEQ ID NO: 2; and
(b3) a protein having an amino acid sequence having at least 98% identity with the amino acid sequence represented by SEQ ID NO: 2, in which an amino acid at a position corresponding to position 117 of the amino acid sequence represented by SEQ ID NO: 2 is lysine.

The glycated protein having a glycated side chain amino group of lysine at a position corresponding to position 117 of the amino acid sequence represented by SEQ ID NO: 2 of the protein according to any one of (b1) to (b3) above is also referred to as "the glycated protein according to the second embodiment of the present invention".

In the amino acid sequence of the glycated protein according to the second embodiment of the present invention, an amino acid at a position corresponding to position 117 of the amino acid sequence represented by SEQ ID NO: 2 is lysine. The glycated protein according to the second embodiment of the present invention is a glycated protein having a glycated side chain amino group of lysine at a position corresponding to position 117 of the amino acid sequence represented by SEQ ID NO: 2.

In the glycated protein according to the second embodiment of the present invention, a sugar bound to a side chain amino group of lysine and its preferred embodiments are as described above for the glycated protein according to the first embodiment.

The position in the amino acid sequence which corresponds to lysine at position 117 of the amino acid sequence represented by SEQ ID NO: 2 is also referred to as a "position corresponding to position 117 of SEQ ID NO: 2".

Lysine at a position corresponding to position 117 of the amino acid sequence represented by SEQ ID NO: 2 can be easily identified in any of the proteins (polypeptides) (b1), (b2), and (b3) above. When identifying lysine at a position corresponding to position 117 of the amino acid sequence of SEQ ID NO: 2 in any of the proteins (polypeptides) (b1), (b2), and (b3) above, identification can be made by the same method as in the case of the glycated protein according to the first embodiment of the present invention, using a known sequence comparison program.

The amino acid sequence represented by SEQ ID NO: 2 is an amino acid sequence of barley-derived protein SerpinZ7 (aliases: BSZ7, SerpinZ7, and HorvuZ7).

In the protein according to (b1) above, the position corresponding to position 117 of SEQ ID NO: 2 is position 117 of the amino acid sequence thereof.

The number of amino acids deleted, substituted, inserted, and/or added in the amino acid sequence of the protein according to (b2) above is preferably 1 to 8, 1 to 7, or 1 to 6, more preferably 1 to 5, still more preferably 1 to 4, particularly preferably 1 to 3, most preferably 1 or 2.

In one embodiment, the protein (b2) is preferably a protein according to any one of (b21) to (b24) below. The proteins according to (b21) to (b24) are SerpinZ7 derived from barley (binomial name: *Hordeum vulgare*).
(b21) A protein having the amino acid sequence represented by SEQ ID NO: 2 in which position 173 is alanine
(b22) A protein having the amino acid sequence represented by SEQ ID NO: 2 in which position 292 is threonine
(b23) A protein having the amino acid sequence represented by SEQ ID NO: 2 in which position 303 is glutamic acid
(b24) A protein having the amino acid sequence represented by SEQ ID NO: 2 in which position 325 is leucine

Preferably, the amino acid sequence of the protein (b3) above has at least 99% identity with the amino acid sequence represented by SEQ ID NO: 2.

In one embodiment, the glycated protein according to the second embodiment of the present invention is preferably a glycated protein having a glycated side chain amino group of lysine at a position corresponding to position 117 of the amino acid sequence represented by SEQ ID NO: 2 of the protein according to (b1) above (protein having the amino acid sequence represented by SEQ ID NO: 2). The glycated protein can also be referred to as a glycated protein having an amino acid sequence represented by SEQ ID NO: 2 and having a glycated side chain amino group of lysine at position 117 of the amino acid sequence.

Each of the proteins (b1), (b2), and (b3) may have a glycated side chain amino group of lysine other than lysine at a position corresponding to position 117 of the amino acid sequence represented by SEQ ID NO: 2.

The production process and source of the glycated protein according to the second embodiment of the present invention are not limited. In one embodiment, the glycated protein according to the second embodiment of the present invention is preferably a grain-derived protein having a glycated side chain amino group of lysine at a position corresponding to position 117 of the amino acid sequence of SEQ ID NO: 2. Examples of grains include barley, wheat, corn, and rice. Of these, barley is preferred. Preferably, the proteins (b1) to (b3) are barley-derived proteins.

Hereinafter, the glycated protein according to the first embodiment of the present invention and the glycated protein according to the second embodiment of the present invention are also collectively referred to as "the glycated protein of the present invention".

The method of producing the glycated protein of the present invention is not limited. For example, the glycated protein of the present invention can be purified from grains, beer brewed from grains used as raw materials, or the like. The grains are preferably barley grains, more preferably barley malt grains. In one embodiment, preferred malt for use is North American malt.

Any method may be used to obtain a glycated protein from beer. A usual isolation/purification method can be used. Examples of the isolation/purification method include ammonium sulphate precipitation, gel filtration chromatography, ion exchange chromatography, affinity chromatography, reversed phase high performance liquid chromatography, dialysis, and ultrafiltration. These methods can be used alone or in appropriate combination. For example, using a method described in Examples, beer is subjected to column chromatography using a cation-exchange resin to collect a fraction containing a 40 kDa protein, ammonium sulphate is added to the fraction for salting out, and the supernatant is collected, whereby a solution (the supernatant) containing a glycated protein can be obtained. The solution is concentrated by a known method, whereby a purified product of the glycated protein of the present invention can be obtained.

Glycation of an amino group of lysine in the resulting protein can be confirmed by colorimetric quantification of the glycated lysine by the nitroblue tetrazolium (NBT) method.

In the glycated protein according to the first embodiment of the present invention, glycation of a side chain amino group of at least one of lysine at a position corresponding to position 160 or lysine at a position corresponding to position 189 of the amino acid sequence represented by SEQ ID NO: 1 can be confirmed by fragmenting the purified protein by a known method and analyzing the resulting peptide fragments by LC-MS/MS.

In the glycated protein according to the second embodiment of the present invention, glycation of a side chain amino group of lysine at a position corresponding to position 117 of the amino acid sequence represented by SEQ ID NO: 2 can be confirmed by fragmenting the purified protein by a known method and analyzing the resulting peptide fragments by LC-MS/MS.

As shown in Examples described later, the glycated protein having a glycated side chain amino group of at least one of lysine at a position corresponding to position 160 or lysine at a position corresponding to position 189 of the amino acid sequence represented by SEQ ID NO: 1 had an excellent rich flavor imparting effect. The glycated protein having a glycated side chain amino group of lysine at a position corresponding to position 117 of the amino acid sequence represented by SEQ ID NO: 2 had an excellent rich flavor imparting effect.

The glycated protein of the present invention is useful for imparting a rich flavor to a food or beverage. When the glycated protein of the present invention is added to a food or beverage, a richer flavor can be imparted when the proportion of the protein is higher.

In the present invention, the term "rich flavor" refers to the sense of taste that cannot be expressed by the five basic tastes, i.e., sweet, salty, sour, bitter, and umami. It is the sense of taste expressed by the intensity of the taste (powerfulness), spread of the taste, thickness of the taste, changes in taste over time (lingering taste or persistence of the taste), and the like. In the present invention, the phrase "imparting a rich flavor" includes, for example, imparting a rich flavor to a food or beverage not having a rich flavor and enhancing the rich flavor of a food or beverage having a rich flavor. The presence or degree of the rich flavor can be evaluated by sensory evaluation.

The glycated protein of the present invention can be used by being added to a food or beverage. The amount of the glycated protein of the present invention in a food or beverage is not limited. The amount can be appropriately set depending on the type of food or beverage, desired level of rich flavor, and the like. In one embodiment, the glycated protein of the present invention can be suitably used for imparting a rich flavor to a beer-taste alcoholic beverage, non-alcoholic beer-taste beverage, or the like. The beer-taste alcoholic beverage means an alcoholic beverage having the characteristic aroma of beer which is produced when the beer is brewed by a usual method. The non-alcoholic beer-taste beverage means a carbonated beverage having a beer-like flavor. It is a non-alcoholic beverage substantially free of alcohol. The glycated protein of the present invention may be of one type used alone or may be of two or more types used in combination. The glycated protein of the present invention may be a purified protein.

The glycated protein of the present invention can be used as an active ingredient of a rich flavor imparting agent for use in imparting a rich flavor to a food or beverage or the like.

The present invention also encompasses a rich flavor imparting agent containing at least one of the glycated protein according to the first embodiment of the present invention (glycated protein (A)) or the glycated protein according to the second embodiment of the present invention (glycated protein (B)).

The rich flavor imparting agent of the present invention contains at least one of the followings: a glycated protein having a glycated side chain amino group of at least one of lysine at a position corresponding to position 160 or lysine at a position corresponding to position 189 of an amino acid sequence represented by SEQ ID NO: 1 of the protein according to any one of (a1) to (a3) above; or a glycated protein having a glycated side chain amino group of lysine at a position corresponding to position 117 of an amino acid sequence represented by SEQ ID NO: 2 of the protein according to any one of (b1) to (b3) above.

The rich flavor imparting agent of the present invention contains at least one of the glycated proteins of the present invention, and usually contains at least one of the glycated proteins as an active ingredient. In one embodiment, preferably, the rich flavor imparting agent contains the glycated protein according to the first embodiment and the glycated protein according to the second embodiment of the present invention. The rich flavor imparting agent may contain the glycated protein according to the first embodiment of the present invention or the glycated protein according to the second embodiment of the present invention. The glycated protein according to the first embodiment of the present invention may be of one type used alone or may be of two or more types used in combination. The glycated protein according to the second embodiment of the present invention may be of one type used alone or may be of two or more types used in combination. The glycated protein according to the first embodiment of the present invention, the glycated protein according to the second embodiment of the present invention, and preferred embodiments thereof are as described above.

Preferably, the rich flavor imparting agent contains at least one of the followings: a glycated protein (glycated protein (a1)) having a glycated side chain amino group of at least one of lysine at a position corresponding to position 160 or lysine at a position corresponding to position 189 of an amino acid sequence represented by SEQ ID NO: 1 of the protein according to (a1); or a glycated protein (glycated protein (b1)) having a glycated side chain amino group of lysine at a position corresponding to position 117 of an amino acid sequence represented by SEQ ID NO: 2 of the protein according to (b1). More preferably, the rich flavor imparting agent contains the glycated protein (a1) and the glycated protein (b1).

The rich flavor imparting agent of the present invention can be used, for example, for imparting a rich flavor to a food or beverage. The rich flavor imparting agent of the present invention can be suitably used, for example, for imparting a rich flavor to a beer-taste alcoholic beverage or a non-alcoholic beer-taste beverage. The beer-taste alcoholic beverage is preferably beer. When imparting a rich flavor to a food or beverage by the rich flavor imparting agent of the present invention, the rich flavor imparting agent is simply added to the food or beverage. The rich flavor imparting agent may be added to a food or beverage by any method, such as premixing the rich flavor imparting agent with raw materials to be used in production or adding the rich flavor imparting agent at any step in the food or beverage production process.

The present invention also encompasses use of at least one of the glycated protein according to the first embodiment of the present invention (glycated protein (A)) or the glycated protein according to the second embodiment of the present invention (glycated protein (B)) for imparting a rich flavor to a food or beverage.

Adding at least one of the glycated protein according to the first embodiment of the present invention (glycated protein (A)) or the glycated protein according to the second embodiment of the present invention (glycated protein (B)) to a food or beverage can impart a rich flavor to the food or beverage.

The present invention also encompasses a method of imparting a rich flavor to a food or beverage, the method including adding at least one of the glycated protein according to the first embodiment or the glycated protein according to the second embodiment of the present invention to a food or beverage.

The use and the method use at least one of the followings: a glycated protein having a glycated side chain amino group of at least one of lysine at a position corresponding to position 160 or lysine at a position corresponding to position 189 of an amino acid sequence represented by SEQ ID NO: 1 of the protein according to any one of (a1) to (a3) above; or a glycated protein having a glycated side chain amino group of lysine at a position corresponding to position 117 of an amino acid sequence represented by SEQ ID NO: 2 of the protein according to any one of (b1) to (b3) above.

The glycated protein of the present invention may be added to a food or beverage by any method, such as premixing the glycated protein with raw materials to be used in production or adding the glycated protein at any step in the food or beverage production process. In the method of imparting a rich flavor to a food or beverage, the rich flavor imparting agent can be added to a food or beverage. Preferably, the food or beverage is a beer-taste alcoholic beverage or a non-alcoholic beer-taste beverage. The use and the method use at least one of the glycated proteins of the present invention. The glycated protein according to the first embodiment of the present invention may be of one type used alone or may be of two or more types used in combination. The glycated protein according to the second embodiment of the present invention may be of one type used alone or may be of two or more types used in combination.

In one embodiment, preferably, the use and the method use the glycated protein according to the first embodiment of the present invention and the glycated protein according to the second embodiment of the present invention. The use and the method may use the glycated protein according to the first embodiment of the present invention or the glycated protein according to the second embodiment of the present invention. In one embodiment, preferably, the use and the method use at least one of the glycated protein
(a1) or the glycated protein (b1). More preferably, the use and the method use the glycated protein (a1) and the glycated protein (b1).

### EXAMPLES

The present invention is specifically described below with reference to examples. The present invention is not limited to these examples.

### <Example 1>

North American malt was crushed to an appropriate particle size, introduced into a mashing tank, and then mixed with warm water. The mixture was kept at a temperature suitable for a malt enzyme for a period of time sufficient for saccharification to convert starch into a sugar by the action of the malt enzyme, whereby a saccharified liquid was produced. Subsequently, the saccharified liquid was filtered, and hop was added to the resulting filtrate, followed by boiling, whereby hot wort was produced for use for fermentation. The hot wort was cooled, and beer yeast was added thereto. The sugar in the wort was broken down into alcohol and carbon dioxide by the action of the yeast during a few days, whereby newly fermented beer was produced. The newly fermented beer was transferred to a beer storage tank, and stored at a low temperature for several tens of days. During this period, the beer was aged, and the taste and aroma of the beer were harmonized. After aging, the beer was filtered and bottled.

A glycated protein was purified as described below from the beer brewed from North American malt (hereinafter also referred to as "beer (I)") by the method described above.

### (1) Fractionation by cation-exchange resin

A cation-exchange resin "SP Sepharose" (50 mL) was placed in an empty column.

The beer (I) was adsorbed onto the resin. Subsequently, the resin used for adsorption was transferred to another column, washed with a 20 mM sodium acetate buffer (pH 4.5), and then eluted with 20 mM sodium acetate (pH 4.5) + 0.5 M-NaCl, whereby fractions were collected. The resulting fractions were evaluated by SDS-PAGE, and fractions containing a 40 kDa glycated protein (40 kDa protein) were collected as cation-exchange resin-bound fractions.

### (2) Ultrafiltration (buffer replacement)

The cation-exchange resin-bound fractions obtained in (1) were added to an ultrafiltration unit (Amicon Ultra-15 30K available from Merck) washed with water, centrifuged (3500 rpm, 10 min, room temperature), and ultrafiltered, whereby a concentrate was obtained.

### (3) Ammonium sulfate fractionation

To a beaker were added a 20 mM phosphate buffer (pH 7.0) and 2 M ammonium sulphate, and the concentrate obtained in (2) was added dropwise to the beaker, followed by stirring. Then, the resulting suspension was centrifuged (2330 g, 10 min, room temperature). The supernatant was collected in a different container. The collected solution was concentrated in an ultrafiltration unit. To the concentrate was added 20 mM sodium acetate (pH 4.5), followed by centrifugation (2330 g, 10 min, room temperature) for concentration, whereby a purified product of the 40 kDa protein (Bradford quantification (bovine serum albumin (BSA) equivalent), 20.4 mg/mL, 2.21 mL) was obtained. The purity of the resulting purified product of the 40 kDa protein was confirmed by SDS-PAGE.

### Analysis of 40 kDa protein and its modification

The 40 kDa protein was analyzed as follows.

### (1) Preparation of gel piece

A purified 40 kDa protein solution (concentration 1 mg/mL, 20 mM sodium acetate buffer (pH 4.5), 10 µL) was electrophoresed by SDS-PAGE and stained with coomassie brilliant blue (CBB). Subsequently, a gel of the 40 kDa protein was cut out.

### (2) Enzyme digestion of protein

The cut-out gel piece was sliced, followed by reduction with dithiothreitol (56°C, 1 hr) and carbamide methylation with iodoacetamide (room temperature under light-shielded conditions, 45 min). Then, a 0.01% ProteaseMax-containing 10 ng/µL chymotrypsin solution (5 mM calcium chloride, 50 mM ammonium bicarbonate solution) (15 µL), 5 mM calcium chloride, and a 50 mM ammonium bicarbonate solution (15 µL) were added, followed by overnight incubation. Subsequently, the resulting enzyme digestion solution was collected. The collected solution was solidified by drying in vacuum, which was then redissolved in a 0.1% formic acid solution.

The resulting product was subjected to LC-MS/MS analysis.

### (3) LC-MS/MS measurement

LC-MS/MS measurement was performed under the following conditions.
Device used: direct flow type nano LC system "Easy-nLC 1000^{™}" (Thermo Fisher Scientific)
Trap column: Acclaim PepMap^{®} (Thermo Fisher Scientific)
Analysis column: Nano HPLC Capillary Column (Nikkyo Technos Co., Ltd.)
Liquid chromatograph mass spectrometer: Q Exactive Plus (Thermo Fisher Scientific)
Mobile phase: solvent A: 0.1% formic acid/water; solvent B: 0.1% formic acid/acetonitrile
Flow rate: 300 nL/min
Gradient: 0-40% B/0-30 min, 40-60% B/30-35 min, 60-90% B/35-37 min, 90% B/37-45 min
Amount introduced: 10 µL
Ionization mode: ESI Positive
Measurement range: MS1 (m/z 350-1750)
Data dependent scan mode

### (4) Analysis of protein and modification

The protein was identified and modification was searched under the following conditions.
Search software: Proteome Discoverer 2.2.0.388 (available from Thermo Fisher Scientific)
Species: barley (*Hordeum vulgare*), hop (*Humulus*), yeast *(Saccharomyces cerevisiae)*
Search conditions: digestive enzyme: Chymotrypsin
Modification (Static): Carbamidomethyl (Cysteine)
Modification (Dynamic): Oxidation (Methionine), Hex (K, R, Protein N-term), Hex(2) (K, R, Protein N-term), Hex(3) (K, R, Protein N-term), Acetyl (Protein N-term)
Precursor ion mass error range: Monoisotopic, ± 10 ppm Product ion mass error range: ± 0.02 Da
Maximum number of missed cleavages: 5
Confidence level (Percolator): High (level with the highest confidence of the three levels of confidence)
Database: SwissProt

The results show that the 40 kDa glycated protein includes glycated barley SerpinZ4 (sequence coverage: 77.2%) and glycated barley SerpinZ7 (sequence coverage: 72.8%). The amino acid sequence of the barley SerpinZ4 is represented by SEQ ID NO: 1, and the amino acid sequence of the barley SerpinZ7 is represented by SEQ ID NO: 2.

Tables 1 to 8 show the results of LC-MS/MS analysis of modification of the barley SerpinZ4.

Tables 1 to 4 show the results of LC-MS/MS analysis of a peptide fragment (EAVGQVNSWVEQVTTGLIKQILPPGSVDNTTKL (SEQ ID NO: 3)) (Z4 (142-174) peptide) consisting of amino acids at positions 142 to 174 of an amino acid sequence (amino acid sequence represented by SEQ ID NO: 1) of the barley SerpinZ4 purified from the beer.

Table 1 shows m/z values of precursor ions of the peptide fragment, as obtained by LC-MS. Tables 2 to 4 show m/z values of fragment ions of the peptide fragment shown in Table 1, as obtained by LC-MS/MS.

Table 2 shows the results of analysis of fragment ions of MH⁺ 3521.885 Da peptide shown in Table 1. The fragment ions of MH⁺ 3521.885 Da peptide are fragment ions of an unglycated Z4 (142-174) peptide. Table 3 shows the results of analysis of fragment ions of MH⁺ 3683.935 Da peptide shown in Table 1. Table 4 shows the results of analysis of fragment ions of MH⁺ 3845.980 Da peptide shown in Table 1.

Tables 5 to 8 show the results of LC-MS/MS analysis of a peptide fragment (FKGAWDQKFDESNTKCDSF (SEQ ID NO: 4)) (Z4 (182-200) peptide) consisting of amino acids at positions 182 to 200 of the amino acid sequence of the barley SerpinZ4 purified from the beer. Table 5 shows m/z values of precursor ions of the peptide fragment, as obtained by LC-MS. Tables 6 to 8 show m/z values of fragment ions of the peptide fragment, as obtained by LC-MS/MS. Table 6 shows the results of analysis of fragment ions of MH⁺ 2310.012 Da peptide shown in Table 5. The fragment ions of MH⁺ 2310.012 Da peptide are fragment ions of an unglycated Z4 (182-200) peptide. Table 7 shows the results of analysis of fragment ions of MH⁺ 2472.064 Da peptide shown in Table 5. Table 8 shows the results of analysis of fragment ions of MH⁺ 2634.118 Da peptide shown in Table 5.

In Tables 2 to 4 and 6 to 8, "No." indicates the amino acid number in the amino acid sequence represented by SEQ ID NO: 1. Bold numbers in cells with bold lines represent detected fragment ions. K19 in modification in the peptides in Table 1 shows detection of modification of lysine at position 19 of the Z4 (142-174) peptide. K8 in modification in the peptides in Table 5 shows detection of modification of lysine at position 8 of the Z4 (182-200) peptide.

**[Table 1]**

| Sequences | Modification in peptides | Charge | Monoisotopic m/z | MH+ |
|---|---|---|---|---|
| EAVGQVNSWVEQVTTGLIKQILPPGSVDNTTKL | | 3 | 1174.633 | 3521.885 |
| EAVGQVNSWVEQVTTGLI**k(Hex)**QILPPGSVDNTTKL | K19-Hex (162.05282 Da) | 4 | 921.739 | 3683.935 |
| EAVGQVNSWVEQVTTGLI**k(di-Hex)**QILPPGSVDNTTKL | K19-diHex (324.10560 Da) | 3 | 1282.665 | 3845.980 |

**[Table 2]**

| No. | b+ | b2+ | Seq | y+ | y2+ |
|---|---|---|---|---|---|
| 142 | 130.050 | 65.529 | E | | |
| 143 | 201.087 | 101.047 | A | 3392.842 | **1696.925** |
| 144 | **300.155** | 150.581 | V | 3321.805 | 1661.406 |
| 145 | **357.177** | 179.092 | G | 3222.737 | 1611.872 |
| 146 | **485.235** | 243.121 | Q | 3165.715 | 1583.361 |
| 147 | **584.304** | 292.656 | V | 3037.657 | **1519.332** |
| 148 | **698.347** | 349.677 | N | 2938.588 | **1469.798** |
| 149 | **785.379** | 393.193 | S | 2824.545 | **1412.776** |
| 150 | **971.458** | **486.233** | W | 2737.513 | **1369.260** |
| 151 | **1070.527** | 535.767 | V | 2551.434 | **1276.221** |
| 152 | 1199.569 | 600.288 | E | 2452.366 | **1226.687** |
| 153 | 1327.628 | 664.317 | Q | 2323.323 | **1162.165** |
| 154 | 1426.696 | 713.852 | V | 2195.265 | **1098.136** |
| 155 | 1527.744 | 764.376 | T | **2096.196** | **1048.602** |
| 156 | 1628.791 | 814.899 | T | 1995.1481 | 998.078 |
| 157 | 1685.813 | **843.410** | G | **1894.101** | 947.554 |
| 158 | 1798.897 | 899.952 | L | 1837.079 | 919.043 |
| 159 | 1911.981 | 956.494 | I | **1723.995** | 862.501 |
| 160 | 2040.076 | 1020.542 | **K** | **1610.911** | 805.959 |
| 161 | 2168.135 | 1084.571 | Q | **1482.816** | 741.912 |
| 162 | 2281.219 | 1141.113 | I | **1354.758** | 677.882 |
| 163 | 2394.303 | 1197.655 | L | **1241.674** | 621.340 |
| 164 | 2491.356 | 1246.181 | P | **1128.590** | **564.798** |
| 165 | 2588.408 | 1294.708 | P | **1031.537** | 516.272 |
| 166 | 2645.430 | 1323.219 | G | **934.484** | 467.746 |
| 167 | 2732.462 | 1366.735 | S | **877.463** | 439.235 |
| 168 | 2831.530 | 1416.269 | V | **790.431** | 395.719 |
| 169 | 2946.557 | 1473.782 | D | **691.362** | 346.185 |
| 170 | 3060.600 | 1530.804 | N | **576.335** | 288.671 |
| 171 | 3161.648 | 1581.328 | T | **462.292** | 231.650 |
| 172 | 3262.695 | 1631.851 | T | **361.245** | 181.126 |
| 173 | 3390.790 | 1695.899 | K | **260.197** | 130.602 |
| 174 | | | L | 132.1021 | 66.555 |

**[Table 3]**

| No. | b+ | b2+ | b3+ | Seq | y+ | y2+ |
|---|---|---|---|---|---|---|
| 142 | 130.0499 | 65.529 | 44.021 | E | | |
| 143 | 201.0870 | 101.047 | 67.701 | A | 3554.895 | 1777.951 |
| 144 | **300.155** | 150.581 | 100.723 | V | 3483.858 | 1742.433 |
| 145 | **357.177** | 179.092 | 119.730 | G | 3384.790 | 1692.898 |
| 146 | **485.235** | 243.121 | 162.417 | Q | 3327.768 | 1664.388 |
| 147 | **584.304** | 292.656 | 195.439 | V | 3199.710 | 1600.358 |
| 148 | **698.347** | 349.677 | 233.454 | N | 3100.641 | 1550.824 |
| 149 | **785.379** | 393.193 | 262.464 | S | 2986.598 | 1493.803 |
| 150 | **971.458** | **486.233** | 324.491 | W | 2899.566 | 1450.287 |
| 151 | **1070.527** | **535.767** | 357.514 | V | 2713.487 | 1357.247 |
| 152 | **1199.569** | **600.288** | 400.528 | E | 2614.419 | 1307.713 |
| 153 | **1327.628** | **664.317** | 443.214 | Q | 2485.376 | 1243.192 |
| 154 | **1426.696** | **713.852** | 476.237 | V | 2357.317 | 1179.162 |
| 155 | 1527.7438 | 764.376 | 509.919 | T | 2258.249 | 1129.628 |
| 156 | 1628.7915 | 814.899 | 543.602 | T | 2157.201 | 1079.104 |
| 157 | 1685.8129 | **843.410** | 562.609 | G | 2056.154 | 1028.580 |
| 158 | 1798.897 | 899.952 | **600.304** | L | 1999.132 | 1000.070 |
| 159 | 1911.981 | 956.494 | 637.999 | I | 1886.048 | 943.528 |
| 160 | 2202.129 | 1101.568 | 734.714 | **K-Hex** | 1772.964 | 886.986 |
| 161 | 2330.187 | 1165.597 | 777.401 | Q | **1482.816** | 741.912 |
| 162 | 2443.272 | 1222.139 | 815.095 | I | **1354.758** | 677.882 |
| 163 | 2556.356 | 1278.681 | 852.790 | L | **1241.674** | 621.340 |
| 164 | 2653.408 | 1327.208 | 885.141 | P | **1128.590** | **564.798** |
| 165 | 2750.461 | 1375.734 | 917.492 | P | **1031.537** | **516.272** |
| 166 | 2807.483 | 1404.245 | 936.499 | G | **934.484** | 467.746 |
| 167 | 2894.515 | 1447.761 | 965.510 | S | **877.463** | 439.235 |
| 168 | 2993.583 | 1497.295 | 998.533 | V | **790.431** | 395.719 |
| 169 | 3108.610 | 1554.809 | 1036.875 | D | **691.362** | 346.185 |
| 170 | 3222.653 | 1611.830 | 1074.889 | N | **576.335** | 288.671 |
| 171 | 3323.701 | 1662.354 | 1108.572 | T | 462.292 | 231.650 |
| 172 | 3424.748 | 1712.878 | 1142.254 | T | **361.245** | 181.126 |
| 173 | 3552.843 | 1776.925 | 1184.953 | K | **260.197** | 130.602 |
| 174 | | | | L | 132.102 | 66.555 |

**[Table 4]**

| No. | b+ | b2+ | Seq | y+ | y2+ |
|---|---|---|---|---|---|
| 142 | 130.050 | 65.529 | E | | |
| 143 | 201.087 | 101.047 | A | 3716.948 | 1858.978 |
| 144 | **300.155** | 150.581 | V | 3645.911 | 1823.459 |
| 145 | **357.177** | 179.092 | G | 3546.842 | 1773.925 |
| 146 | **485.235** | 243.121 | Q | 3489.821 | 1745.414 |
| 147 | **584.304** | 292.656 | V | 3361.762 | 1681.385 |
| 148 | **698.347** | 349.677 | N | 3262.694 | 1631.851 |
| 149 | **785.379** | 393.193 | S | 3148.651 | 1574.829 |
| 150 | **971.458** | **486.233** | W | 3061.619 | 1531.313 |
| 151 | **1070.527** | 535.767 | V | 2875.540 | 1438.274 |
| 152 | 1199.569 | 600.288 | E | 2776.471 | 1388.739 |
| 153 | 1327.628 | 664.317 | Q | 2647.429 | 1324.218 |
| 154 | 1426.696 | 713.852 | V | 2519.370 | 1260.189 |
| 155 | 1527.744 | 764.376 | T | 2420.302 | 1210.655 |
| 156 | 1628.791 | 814.899 | T | 2319.254 | 1160.131 |
| 157 | 1685.813 | **843.410** | G | 2218.206 | 1109.607 |
| 158 | 1798.897 | 899.952 | L | 2161.185 | 1081.096 |
| 159 | 1911.981 | 956.494 | I | 2048.101 | 1024.554 |
| 160 | 2364.182 | 1182.594 | **K-diHex** | 1935.017 | 968.012 |
| 161 | 2492.240 | 1246.624 | Q | **1482.816** | 741.912 |
| 162 | 2605.324 | 1303.166 | I | **1354.758** | 677.882 |
| 163 | 2718.408 | 1359.708 | L | **1241.674** | 621.340 |
| 164 | 2815.461 | 1408.234 | P | **1128.590** | **564.798** |
| 165 | 2912.514 | 1456.761 | P | **1031.537** | 516.272 |
| 166 | 2969.535 | 1485.271 | G | **934.484** | 467.746 |
| 167 | 3056.567 | 1528.787 | S | **877.463** | 439.235 |
| 168 | 3155.636 | 1578.322 | V | **790.431** | 395.719 |
| 169 | 3270.663 | 1635.835 | D | **691.362** | 346.185 |
| 170 | 3384.706 | 1692.856 | N | **576.335** | 288.671 |
| 171 | 3485.753 | 1743.380 | T | **462.292** | 231.650 |
| 172 | 3586.801 | 1793.904 | T | **361.245** | 181.126 |
| 173 | 3714.896 | 1857.952 | K | 260.197 | 130.602 |
| 174 | | | L | 132.102 | 66.555 |

**[Table 5]**

| Sequences | Modification in peptides | Charge | Monoisotopic m/z | MH+ |
|---|---|---|---|---|
| FKGAWDQKFDESNTKCDSF | | 2 | 1155.509 | 2310.012 |
| FKGAWDQk(Hex)FDESNTKCDSF | K8-Hex (162.05282 Da) | 3 | 824.693 | 2472.064 |
| FKGAWDQk(di-Hex)FDESNTKCDSF | K8-diHex (324.10560 Da) | 2 | 1317.563 | 2634.118 |

**[Table 6]**

| No. | b+ | b2+ | Seq | y+ | y2+ |
|---|---|---|---|---|---|
| 182 | 148.076 | 74.541 | F | | |
| 183 | **276.171** | 138.589 | K | 2162.945 | **1081.976** |
| 184 | **333.192** | 167.100 | G | **2034.850** | **1017.929** |
| 185 | **404.229** | 202.618 | A | **1977.828** | 989.418 |
| 186 | **590.309** | 295.658 | W | **1906.791** | **953.899** |
| 187 | **705.335** | 353.171 | D | **1720.712** | **860.860** |
| 188 | **833.394** | 417.201 | Q | **1605.685** | **803.346** |
| 189 | **961.489** | 481.248 | K | **1477.626** | **739.317** |
| 190 | **1108.557** | 554.782 | F | **1349.531** | 675.269 |
| 191 | **1223.584** | **612.296** | D | **1202.463** | 601.735 |
| 192 | **1352.627** | 676.817 | E | **1087.436** | 544.222 |
| 193 | **1439.659** | 720.333 | S | **958.393** | 479.700 |
| 194 | **1553.702** | **777.355** | N | **871.361** | 436.184 |
| 195 | **1654.750** | **827.878** | T | **757.319** | 379.163 |
| 196 | **1782.845** | **891.926** | K | **656.271** | 328.639 |
| 197 | 1942.875 | **971.941** | C-Carbamido methyl | **528.176** | 264.592 |
| 198 | 2057.902 | **1029.455** | D | **368.145** | 184.576 |
| 199 | 2144.934 | **1072.971** | S | **253.118** | 127.063 |
| 200 | | | F | **166.086** | 83.547 |

**[Table 7]**

| No. | b+ | b2+ | b3+ | Seq | y+ | y2+ |
|---|---|---|---|---|---|---|
| 182 | 148.0761 | 74.541 | 50.030 | F | | |
| 183 | **276.171** | 138.589 | 92.728 | K | 2324.998 | 1163.002 |
| 184 | **333.192** | 167.100 | 111.736 | G | 2196.903 | 1098.955 |
| 185 | **404.229** | 202.618 | 135.415 | A | 2139.881 | 1070.444 |
| 186 | **590.309** | 295.658 | 197.441 | W | 2068.844 | 1034.926 |
| 187 | **705.335** | 353.171 | 235.783 | D | 1882.765 | 941.886 |
| 188 | **833.394** | 417.201 | 278.470 | Q | 1767.738 | 884.373 |
| 189 | 1123.542 | 562.275 | 375.185 | K-Hex | 1639.679 | 820.343 |
| 190 | 1270.610 | 635.809 | 424.208 | F | **1349.531** | 675.269 |
| 191 | 1385.637 | 693.322 | 462.551 | D | **1202.463** | 601.735 |
| 192 | 1514.680 | 757.844 | 505.565 | E | **1087.436** | 544.222 |
| 193 | 1601.712 | 801.360 | 534.575 | S | **958.393** | 479.700 |
| 194 | 1715.755 | **858.381** | 572.590 | N | **871.361** | 436.184 |
| 195 | 1816.802 | 908.905 | 606.272 | T | **757.319** | 379.163 |
| 196 | 1944.897 | 972.952 | 648.971 | K | **656.271** | 328.639 |
| 197 | 2104.928 | 1052.968 | **702.314** | C-Carbam ido methyl | **528.176** | 264.592 |
| 198 | 2219.955 | 1110.481 | 740.657 | D | **368.145** | 184.576 |
| 199 | 2306.987 | 1153.997 | 769.667 | S | **253.118** | 127.063 |
| 200 | | | | F | 166.086 | 83.547 |

**[Table 8]**

| No. | b+ | b2+ | Seq | y+ | y2+ |
|---|---|---|---|---|---|
| 182 | 148.0761 | 74.541 | F | | |
| 183 | **276.171** | 138.589 | K | 2487.050 | 1244.029 |
| 184 | **333.192** | 167.100 | G | 2358.955 | 1179.981 |
| 185 | **404.229** | 202.618 | A | 2301.934 | 1151.471 |
| 186 | **590.309** | 295.658 | W | 2230.897 | 1115.952 |
| 187 | **705.335** | 353.171 | D | 2044.818 | 1022.912 |
| 188 | **833.394** | 417.201 | Q | 1929.791 | 965.399 |
| 189 | 1285.595 | 643.301 | K-diHex | 1801.732 | 901.370 |
| 190 | 1432.663 | 716.835 | F | **1349.531** | 675.269 |
| 191 | 1547.690 | 774.349 | D | **1202.463** | 601.735 |
| 192 | 1676.733 | 838.870 | E | **1087.436** | 544.222 |
| 193 | **1763.765** | **882.386** | S | **958.393** | 479.700 |
| 194 | 1877.808 | 939.407 | N | **871.361** | 436.184 |
| 195 | 1978.855 | 989.931 | T | **757.319** | 379.163 |
| 196 | 2106.950 | 1053.979 | K | **656.271** | 328.639 |
| 197 | 2266.981 | 1133.994 | C-Carbamid omethyl | **528.176** | 264.592 |
| 198 | 2382.008 | **1191.508** | D | 368.145 | 184.576 |
| 199 | 2469.040 | 1235.024 | S | **253.118** | 127.063 |
| 200 | | | F | 166.086 | 83.547 |

The results in Tables 1 to 8 show the presence of glycated SerpinZ4 having a structure in which an amino group of at least one of lysine at position 160 (K160) or lysine at position 189 (K189) was modified (glycated) with a hexose. The Z4 (142-174) peptide contains lysine at position 160 (K160) of SerpinZ4.

The detected m/z values in the columns for b+, b2+, and y+ of Nos. 160 to 173 in Table 2 and the m/z values in the columns for b+, b2+, and y+ of Nos. 160 to 173 in Table 3 show addition of 162 Da corresponding to a hexose to lysine (K160) of the fragment ion No. 160 of MH⁺ 3683.935 Da peptide. This shows that MH⁺ 3683.935 Da peptide contains a peptide having a sequence structure modified with one hexose added to K160. The m/z values in the columns for b+, b2+, and y+ of Nos. 160 to 173 in Table 2 and the m/z values in the columns for b+, b2+, and y+ of Nos. 160 to 173 in Table 4 show addition of 324 Da corresponding to a dihexose to lysine (K160) of the fragment ion No. 160 of MH⁺ 3845.980 Da peptide. This shows that the fragment ions of MH⁺ 3845.980 Da peptide contain a peptide having a sequence structure modified with a dihexose added to K160.

Tables 6 to 8 show the results of analysis of the Z4 (182-200) peptide. The Z4 (182-200) peptide contains lysine at position 189 (K189) of SerpinZ4.

The m/z values in the columns for b+ and y+ of Nos. 189 to 199 in Table 6 and the m/z values in the columns for b+, b2+, and y+ of Nos. 189 to 199 in Table 7 show addition of 162 Da corresponding to a hexose to lysine (K189) of the fragment ion No. 189 of MH⁺ 2472.064 Da peptide. This shows that the fragment ions of MH⁺ 2472.064 Da peptide contain a peptide having a sequence structure modified with one hexose added to K189.

The m/z values in the columns for b+, b2+, y+ of Nos. 189 to 199 in Table 6 and the m/z values in the columns for b+, b2+, and y+ of Nos. 189 to 199 in Table 8 show addition of 324 Da corresponding to a dihexose to lysine (K189) of the fragment ion No. 189 of MH⁺ 2634.118 Da peptide. This shows that the fragment ions of MH⁺ 2634.118 Da peptide contain a peptide having a sequence structure modified with two hexoses added to K189. There are no existing reports on SerpinZ4 having a glycated amino group of at least one of lysine at position 160 (K160) or lysine at position 189 (K189) of the amino acid sequence represented by SEQ ID NO: 1.

The hexose added to lysine of the glycated SerpinZ4 was assumed to be glucose or xylose. The dihexose was assumed to be maltose or isomaltose.

### <Example 2>

Glycated proteins were purified from three different beers, and the resulting purified glycated proteins were separately added to beer for sensory evaluation. The relation between glycation of at least one of lysine at position 160 or lysine at position 189 of the barley SerpinZ4 and the rich flavor imparting effect was examined.

### (Purification of 40 kDa glycated proteins from three respective beers)

40 kDa glycated proteins were purified from the three respective beers (beer B, beer C, and beer D) as in purification of the protein from the beer (I) in Example 1, whereby glycated protein-containing solutions were obtained.

### (Protein concentration of glycated protein solutions purified from three respective beers, and analysis of the glycated proteins)

The protein concentration and the glycated lysine concentration of each of the glycated protein-containing solutions purified from the three respective beers (B, C, and D) were determined by the following method.

### (Glycated lysine concentration)

The concentration of lysine having a glycated side chain amino group was colorimetrically quantified by the nitroblue tetrazolium (NBT) method, using Fructosamine Assay Kit (ab228558 available from Abcam).

The number of tests was 2 (n = 2) in samples and blank. Average values were used.

The amino group content (nmol/mL) of the glycated lysine in each of the glycated protein-containing solutions purified from the respective beers was determined. The protein concentration (mg/mL) in each of the glycated protein-containing solution purified from the respective beers was also measured by the Bradford method (BSA equivalent).

The amino group content (nmol/mL) of the glycated lysine in each glycated protein-containing solution was divided by the protein concentration (mg/mL) to determine the amino group concentration (nmol/mg-protein) of the glycated lysine per weight of the purified glycated protein.

### (Results of analysis of purified glycated proteins)

Table 9 shows the results of analysis of the amino group concentration and the protein concentration (measured by the Bradford method (BSA equivalent)) of the glycated lysine in each of the glycated protein-containing solutions purified from the three respective beers (B, C, and D).

The amino group concentration of each glycated lysine shown in Table 9 is the amino group concentration of each glycated lysine per weight of the corresponding purified glycated protein.

**[Table 9]**

| Beer | Protein concentration (mg/mL) | Amino group concentration of glycated lysine (nmol/mg-protein) | Mass of protein added to beer A (µg/mL) |
|---|---|---|---|
| B | 3.32 | 603.8 | 28.6 |
| C | 5.35 | 675.4 | 25.5 |
| D | 21.63 | 900.0 | 19.2 |

### (Evaluation of impartation of rich flavor to beer by glycated proteins purified from three respective beers)

The glycated protein-containing solutions purified from the three respective beers (B, C, and D) were separately added to commercially available beer A for sensory evaluation. Here, the glycated protein solutions purified from the respective beers B, C, and D were separately added to the beer A such that the sensory evaluation samples would have the same glycated lysine concentration, whereby sample B, sample C, and sample D were obtained. Sample B contained the glycated protein solution purified from the beer B. Sample C contained the glycated protein solution purified from the beer C. Sample D contained the glycated protein solution purified from the beer D. The commercially available beer A had a total purine concentration of 10.39 mg/100 mL. The total purine concentration was determined by the degradation method by Japan Food Research Laboratories.

### (Sensory evaluation)

The commercially available beer A (control (without addition)) and samples B, C, and D were subjected to sensory evaluation. The sensory evaluation was scored in increments of 0.1 points by four expert panelists, with a reference point of 1.5 for the rich flavor of the commercially available beer A without addition of any glycated protein. The scores were averaged out.

Criteria for the rich flavor were as follows.
0 points: No rich flavor was tasted.
1 point: A slight rich flavor was tasted.
2 points: A definite rich flavor was tasted.
3 points: A very strong rich flavor was tasted.

Table 10 shows average scores. Control (without addition) in Table 10 is the beer A to which no glycated protein was added. As a result, as shown in Table 10, the intensity of the rich flavor was the highest in sample C, followed by sample D and sample B.

**[Table 10]**

| Sample | Control (without addition) | B | C | D |
|---|---|---|---|---|
| Intensity of the rich flavor (points) | 1.500 | 1.850 | 2.150 | 2.075 |

(Analysis of the amount of lysine at position 160 and lysine at position 189 each having a sugar bound to its side chain amino group in each of the glycated proteins purified from three respective beers)

Modification of each of the glycated proteins purified from the three respective beers (B, C, and D) was analyzed by the same method as in Example 1. The following areas were calculated: the area of ions obtained by LC-MS from an unglycated peptide fragment (EAVGQVNSWVEQVTTGLIKQILPPGSVDNTTKL (SEQ ID NO: 3), unmodified Z4 (142-174) peptide) consisting of amino acids at positions 142 to 174 of the amino acid sequence of SerpinZ4 (SEQ ID NO: 1); and the area of ions obtained by LC-MS from the peptide fragment (glycated Z4 (142-174) peptide) containing lysine at position 160 (K160) having a sugar added to its side chain amino group. Further, based on these areas, the ratio of (sum of areas of glycated Z4 (142-174) peptide ions)/(area of unmodified Z4 (142-174) peptide ions) was determined for each of the glycated proteins purified from the three respective beers. Then, the percentage of the glycated Z4 (142-174) peptide having a glycated side chain amino group of K160 relative to the unmodified Z4 (142-174) peptide was compared (FIG. 1). (Taking into account that the efficiency of LC-MS ionization may vary depending on the unmodified peptide or modified peptide, the area ratio in each of the samples of the three beers was compared side by side.)

FIG. 1 is a graph showing a ratio of ((sum of areas of glycated Z4 (142-174) peptide ions)/(area of unmodified Z4 (142-174) peptide ions)) in each of glycated proteins purified from the three different beers (B, C, and D). Specifically, the "(area of unmodified Z4 (142-174) peptide ions)" is the area of ions obtained by LC-MS from an unmodified peptide fragment (unmodified Z4 (142-174) peptide) consisting of amino acids at positions 142 to 174 of the amino acid sequence of SerpinZ4 (SEQ ID NO: 1); and the "(sum of areas of glycated Z4 (142-174) peptide ions)" is the sum of the areas of ions obtained by LC-MS from a glycated Z4 (142-174) peptide having one hexose or two hexoses (dihexose) bound to K160 of the peptide. The area ratio on the vertical axis of FIG. 1 is the ratio of the areas described above, i.e., (sum of areas of glycated Z4 (142-174) peptide ions)/(area of unmodified Z4 (142-174) peptide ions).

The "sum of areas of glycated Z4 (142-174) peptide ions" is the sum of the area of Z4 (142-174) peptide ions having a monosaccharide (Hex) bound to a side chain of lysine at position 160 and the area of Z4 (142-174) peptide ions having a disaccharide (diHex) bound to a side chain of lysine at position 160. The "area of unmodified Z4 (142-174) peptide ions" is the area of Z4 (142-174) peptide ions having no sugar bound to a side chain of lysine at position 160 (a side chain of K160 is unmodified).

Further, the following areas were calculated: the area of ions obtained by LC-MS from an unglycated peptide fragment (FKGAWDQKFDESNTKc (carbamidomethyl) DSF, unmodified Z4 (182-200) peptide) consisting of amino acids at positions 182 to 200 of the amino acid sequence of SerpinZ4 (SEQ ID NO: 1); and the area of ions obtained by LC-MS from the peptide fragment (glycated Z4 (182-200) peptide) containing lysine at position 160 (K160) having a sugar added to its side chain amino group. Further, based on these areas, the ratio of (sum of areas of glycated Z4 (182-200) peptide ions)/(area of unmodified Z4 (182-200) peptide ions) was determined for each of the glycated proteins purified from the three respective beers. Then, the percentage of the glycated Z4 (182-200) peptide having a glycated side chain amino group of K189 relative to the unmodified Z4 (182-200) peptide was compared (FIG. 2). (Taking into account that the efficiency of LC-MS ionization may vary depending on the unmodified peptide or modified peptide, the area ratio in each of the samples of the three beers was compared side by side.)

FIG. 2 is a graph showing a ratio of ((sum of areas of glycated Z4 (182-200) peptide ions)/(area of unmodified Z4 (182-200) peptide ions)) in each of glycated proteins purified from the three different beers (B, C, and D). Specifically, the "(area of unmodified Z4 (182-200) peptide ions)" is the area of ions obtained by LC-MS from an unmodified peptide fragment (unmodified Z4 (182-200) peptide) consisting of amino acids at positions 182 to 200 of the amino acid sequence of SerpinZ4 (SEQ ID NO: 1); and the "(sum of areas of glycated Z4 (182-200) peptide ions)" is the sum of the areas of ions obtained by LC-MS from a glycated Z4 (182-200) peptide having one hexose or two hexoses bound to lysine at position 189 (K189) of the peptide. The area ratio on the vertical axis of FIG. 2 is the ratio of the areas described above, i.e., (sum of areas of glycated Z4 (182-200) peptide ions)/(area of unmodified Z4 (182-200) peptide ions).

The "sum of areas of glycated Z4 (182-200) peptide ions" is the sum of the area of Z4 (182-200) peptide ions having a monosaccharide (Hex) bound to a side chain of lysine at position 189 and the area of Z4 (182-200) peptide ions having a disaccharide (diHex) bound to the side chain of the lysine. The "area of unmodified Z4 (182-200) peptide ions" is the area Z4 (182-200) peptide ions having no sugar bound to a side chain of lysine at position 189 (a side chain of K189 is unmodified).

As a result, the ratio of the modified peptide fragment (glycated Z4 (142-174) peptide) to the unmodified peptide fragment (unmodified Z4 (142-174) peptide) in the peptide fragment (glycated Z4 (142-174) peptide) containing lysine at position 160 (K160) having a sugar bound to its side chain amino group was the highest in the beer C, followed by the beer D, and the beer B. The ratio of the modified peptide fragment (glycated Z4 (182-200) peptide) to the unmodified peptide fragment (unmodified Z4 (182-200) peptide) in the peptide fragment containing lysine at position 189 (K189) was also the highest in the beer C, followed by the beer D, and the beer B. These results were consistent with the fact that sample C had the highest score for the rich flavor in the sensory evaluation. This shows that there is a relation between the rich flavor and the amount of the glycated protein having a sugar bound to each of a side chain amino group of lysine at position 160 and a side chain amino group of lysine at position 189.

As shown in FIG. 1 and FIG. 2, clearly, the rich flavor was further enhanced by a higher proportion of the glycated protein having a glycated side chain amino group of at least one of lysine at position 160 or lysine at position 189 of the protein having the amino acid sequence of SEQ ID NO: 1. Clearly, the protein having a glycated amino group of at least one lysine at a position corresponding to position 160 or lysine at a position corresponding to position 189 has an excellent rich flavor imparting effect.

<Example 3>

LC-MS/MS target monitoring was performed for detailed analysis of modification of the glycated SerpinZ7.

Samples for LC-MS/MS analysis were prepared by the same method as in Example 1. LC-MS/MS measurement was performed by the same method as in Example 1, except that data dependent scan mode was set to target ion monitoring. The target ions used were m/z 524.788, m/z 605.814, m/z 686.840, and m/z 767.868.

Tables 11 to 15 show the results of LC-MS/MS analysis of modification of the barley SerpinZ7. Tables 11 to 15 show the results of LC-MS/MS analysis of a peptide fragment (SLKPSFQEL (SEQ ID NO: 5)) (Z7 (115-123) peptide) consisting of amino acids at positions 115 to 123 of the amino acid sequence (amino acid sequence represented by SEQ ID NO: 2) of the barley SerpinZ7 purified from the beer.

Table 11 shows m/z values of precursor ions of the peptide fragment, as obtained by LC-MS. Tables 12 to 15 show m/z values of fragment ions of the peptide fragment shown in Table 11, as obtained by LC-MS/MS.

Table 12 shows the results of analysis of fragment ions of MH⁺ 1048.566 Da peptide shown in Table 11. The fragment ions of MH⁺ 1048.566 Da peptide are fragment ions of an unglycated Z7 (115-123) peptide. Table 13 shows the results of analysis of fragment ions of MH⁺ 1210.619 Da peptide shown in Table 11. Table 14 shows the results of analysis of fragment ions of MH⁺ 1372.670 Da peptide shown in Table 11. Table 15 shows the results of analysis of fragment ions of MH⁺ 1534.729 Da peptide shown in Table 11.

**[Table 11]**

| Sequences | Modification in peptides | Charge | Monoisotopic m/z | MH+ |
|---|---|---|---|---|
| SLKPSFQEL | - | 2 | 524.787 | 1048.566 |
| SL**k(Hex)**PSFQEL | K3-Hex (162.05282 Da) | 2 | 605.813 | 1210.619 |
| SL**k(di-Hex)**PSFQEL | K3-diHex (324.10560 Da) | 2 | 686.084 | 1372.670 |
| SL**k(tri-Hex)**PSFQEL | K3-triHex (486.15900 Da) | 2 | 767.868 | 1534.729 |

In Tables 12 to 14, "No." indicates the amino acid number in the amino acid sequence represented by SEQ ID NO: 2. Bold numbers in cells with bold lines represent detected fragment ions. K3 in modification in the peptides in Table 11 shows detection of modification of lysine at position 3 of the Z7 (115-123) peptide.

**[Table 12]**

| No | b+ | b2+ | Seq | y+ | y2+ |
|---|---|---|---|---|---|
| 115 | 88.039 | 44.523 | S | | |
| 116 | **201.123** | **101.065** | L | 961.535 | 481.271 |
| 117 | **329.218** | **165.113** | **K** | **848.451** | 424.729 |
| 118 | **426.271** | 213.639 | P | **720.356** | 360.682 |
| 119 | **513.303** | 257.155 | S | 623.304 | 312.155 |
| 120 | **660.372** | 330.689 | F | 536.271 | 268.639 |
| 121 | **788.430** | 394.719 | Q | 389.203 | 195.105 |
| 122 | **917.473** | 459.240 | E | **261.145** | 131.076 |
| 123 | | | L | **132.102** | 66.555 |

**[Table 13]**

| No | b+ | b2+ | Seq | y+ | y2+ |
|---|---|---|---|---|---|
| 115 | 88.039 | 44.523 | S | | |
| 116 | **201.123** | 101.065 | L | 1123.588 | 562.298 |
| 117 | **491.271** | 246.139 | **K-Hex** | 1010.504 | 505.756 |
| 118 | **588.324** | 294.666 | P | **720.356** | 360.682 |
| 119 | **675.356** | 338.182 | S | 623.304 | 312.155 |
| 120 | **822.424** | 411.716 | F | 536.271 | 268.639 |
| 121 | **950.483** | 475.745 | Q | 389.203 | 195.105 |
| 122 | **1079.526** | **540.266** | E | **261.145** | 131.076 |
| 123 | | | L | **132.102** | 66.555 |

**[Table 14]**

| No | b+ | b2+ | Seq | y+ | y2+ |
|---|---|---|---|---|---|
| 115 | 88.039 | 44.523 | S | | |
| 116 | **201.123** | **101.065** | L | 1285.641 | 643.324 |
| 117 | **653.324** | 327.166 | **K-diHex** | 1172.557 | 586.782 |
| 118 | **750.377** | **375.692** | P | **720.356** | 360.682 |
| 119 | 837.409 | 419.208 | S | 623.304 | 312.155 |
| 120 | 984.477 | 492.742 | F | 536.271 | 268.639 |
| 121 | 1112.536 | 556.772 | Q | **389.203** | 195.105 |
| 122 | 1241.578 | 621.293 | E | **261.145** | 131.076 |
| 123 | | | L | **132.102** | 66.555 |

**[Table 15]**

| No | b+ | b2+ | Seq | y+ | y2+ |
|---|---|---|---|---|---|
| 115 | 88.039 | 44.523 | S | | |
| 116 | **201.123** | 101.065 | L | 1447.694 | **724.351** |
| 117 | 815.377 | 408.192 | **K-triHex** | 1334.610 | 667.809 |
| 118 | 912.430 | 456.719 | P | **720.356** | 360.682 |
| 119 | 999.462 | 500.235 | S | 623.304 | 312.155 |
| 120 | 1146.531 | 573.769 | F | **536.271** | 268.639 |
| 121 | 1274.589 | 637.798 | Q | **389.203** | **195.105** |
| 122 | 1403.632 | 702.319 | E | **261.145** | 131.076 |
| 123 | | | L | **132.102** | 66.555 |

The results in Tables 11 to 15 show the presence of glycated SerpinZ7 having a structure in which an amino group of lysine at position 117 (K117) was modified (glycated) with a hexose. The Z7 (115-123) peptide contains lysine at position 117 (K117) of SerpinZ7.

The detected m/z values in columns for b+, b2+, and y+ in Table 12 and Table 13 show addition of 162 Da corresponding to a hexose to lysine (K117) of the fragment ion No. 117 of MH⁺ 1210.619 Da peptide. This shows that the fragment ions of MH⁺ 1210.619 Da peptide contain a peptide having a sequence structure modified with one hexose added to K117. The detected m/z values in columns for b+, b2+, and y+ in Table 12 and Table 13 show addition of 324 Da corresponding to a dihexose to lysine (K117) of the fragment ion No. 117 of MH⁺ 1372.670 Da peptide. This shows that the fragment ions of MH⁺ 1372.670 Da peptide contain a peptide having a sequence structure modified with a dihexose added to K117. The detected m/z values in columns for b+, b2+, and y+ in Table 12 and Table 13 show addition of 486 Da corresponding to a trihexose to lysine (K117) of the fragment ion No. 117 of MH⁺ 1534.729 Da peptide. This shows that the fragment ions of MH⁺ 1534.729 Da peptide contain a peptide having a sequence structure modified with a trihexose added to K117.

There are no existing reports on SerpinZ7 having a glycated amino group of lysine at position 117 (K117) of the amino acid sequence represented by SEQ ID NO: 2.

The hexose added to lysine of the glycated SerpinZ7 was assumed to be glucose or xylose. The dihexose was assumed to be maltose or isomaltose. The trihexose was assumed to be maltotriose or isomaltotriose.

### <Example 4>

### (Purification of 40 kDa glycated proteins from three respective beers)

40 kDa glycated proteins were purified from three respective beers (beer E, beer F, and beer G) as in purification of the protein from the beer (I) in Example 1, whereby glycated protein-containing solutions were obtained.

### (Protein concentration of glycated protein solutions purified from three respective beers, and analysis of the glycated proteins)

The same methods as in Example 2 were used to determine the protein concentration (mg/mL) of each of the glycated protein-containing solutions purified from the three respective beers (E, F, and G) and the amino group concentration (nmol/mg-protein) of the glycated lysine per weight of the corresponding purified glycated protein.

### (Results of analysis of purified glycated proteins)

Table 16 shows the results of analysis of the amino group concentration and the protein concentration (measured by the Bradford method (BSA equivalent)) of the glycated lysine in each of the glycated protein-containing solutions purified from the three respective beers (E, F, and G).

The amino group concentration of the glycated lysine shown in Table 16 is the amino group concentration of the glycated lysine per weight of the purified glycated protein.

**[Table 16]**

| Beer | Protein concentration (mg/mL) | Amino group concentration of glycated lysine (nmol/mg-protein) | Mass of protein added to beer A (µg/mL) |
|---|---|---|---|
| E | 3.23 | 470.2 | 22.9 |
| F | 6.02 | 454.9 | 23.7 |
| G | 30.35 | 642.9 | 16.8 |

### (Evaluation of impartation of rich flavor to beer by purified glycated proteins purified from three respective beers)

The glycated protein-containing solutions purified from the three respective beers (E, F, and G) were separately added to commercially available beer A for sensory evaluation. Here, the glycated protein solutions purified from the respective beers E, F, and G were separately added to the beer A such that each sensory evaluation sample would have the same glycated lysine concentration, whereby sample E, sample F and sample G were obtained. Sample E contained the glycated protein solution purified from the beer E. Sample F contained the glycated protein solution purified from the beer F. Sample G contained the glycated protein solution purified from the beer G.

### (Sensory evaluation)

Four expert panelists performed sensory evaluation of the commercially available beer A (control (without addition)) and samples E, F, and G to evaluate rich flavor by the same method as in the sensory evaluation of Example 2, except that the sensory evaluation was scored in increments of 0.05 points.

Table 17 shows average scores. Control (without addition) in Table 17 is the beer A to which no glycated protein was added. As a result, as shown in Table 17, the intensity of the rich flavor was the highest in sample F, followed by sample G and sample E.

**[Table 17]**

| Sample | Control (without addition) | E | F | G |
|---|---|---|---|---|
| Intensity of the rich flavor (points) | 1.500 | 1.775 | 2.238 | 2.063 |

### (Analysis of the amount of lysine having a sugar bound to its side chain amino group)

Modification of each of the glycated proteins purified from the three respective beers (E, F, and G) was analyzed by the same method as in Example 1. The following areas were calculated: the area of ions obtained by LC-MS from an unglycated peptide fragment (SLKPSFQEL (SEQ ID NO: 5), unmodified Z7 (115-123) peptide) consisting of amino acids at positions 115 to 123 of the amino acid sequence of SerpinZ7 (SEQ ID NO: 2); and the area of ions obtained by LC-MS from the peptide fragment (glycated Z7 (115-123) peptide) containing lysine at position 117 (K117) having a sugar added to its side chain amino group. Further, based on these areas, the ratio of the sum of the areas of glycated Z7 (115-123) peptide ions to the area of unmodified Z7 (115-123) peptide ions, i.e., (sum of areas of glycated Z7 (115-123) peptide ions)/(area of unmodified Z7 (115-123) peptide ions), was determined for each of the glycated proteins purified from the three respective beers. Then, the percentage of the glycated Z7 (115-123) peptide having a glycated side chain amino group of K117 relative to the unmodified Z7 (115-123) peptide was compared.

### (Taking into account that the efficiency of LC-MS ionization may vary depending on the unmodified peptide or modified peptide, the area ratio in each of the samples of the three beers was compared side by side.)

Table 18 shows the ratio of the sum of the areas of glycated Z7 (115-123) peptide ions to the area of unmodified Z7 (115-123) peptide ions (the ratio of the sum of areas of ions obtained by LC-MS from the glycated Z7 (115-123) peptide to the area of unmodified Z7 (115-123) peptide ions).

The "sum of areas of glycated Z7 (115-123) peptide ions" is the sum of the area of Z7 (115-123) peptide ions having a monosaccharide (Hex) bound to a side chain of lysine at position 117, the area of Z7 (115-123) peptide ions having a disaccharide (diHex) bound to a side chain of the lysine, and the area of Z7 (115-123) peptide ions having a trisaccharide (triHex) bound to a side chain of the lysine. The "area of unmodified Z7 (115-123) peptide ions" is the area of Z7 (115-123) peptide ions having no sugar bound to a side chain of lysine at position 117 (a side chain of K117 is unmodified).

**[Table 18]**

| Beer | Ratio of sum of areas of ions obtained by LC-MS from glycated Z7 (115-123) peptide to the area of unmodified Z7 (115-123) peptide ions |
|---|---|
| E | 0.35 |
| F | 0.59 |
| G | 0.51 |

The ratio of the glycated peptide fragment (glycated Z7 (115-123) peptide) to the unmodified peptide fragment (unmodified Z7 (115-123) peptide) was the highest in the beer F, followed by the beer G, and the beer E. These results were consistent with the fact that sample F had the highest score for the rich flavor in the sensory evaluation. Each of the glycated protein solutions purified from the respective beer E, beer F, and beer G contained the glycated protein having a glycated side chain amino group of at least one of lysine at position 160 or lysine at position 189 of the protein having the amino acid sequence of SEQ ID NO: 1.

### INDUSTRIAL APPLICABILITY

Use of the glycated protein of the present invention makes it possible to impart a rich flavor to a food or

## Claims

1. A glycated protein having a glycated side chain amino group of lysine, the glycated protein comprising:
a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
(a1) a protein having the amino acid sequence represented by SEQ ID NO: 1;
(a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
(a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine.

2. The glycated protein according to claim 1,
wherein the protein according to any one of (a1) to (a3) is a barley-derived protein.

3. A glycated protein having a glycated side chain amino group of lysine, the glycated protein comprising:
a glycated side chain amino group of lysine at a position corresponding to position 117 of an amino acid sequence represented by SEQ ID NO: 2 of a protein according to any one of (b1) to (b3) below:
(b1) a protein having the amino acid sequence represented by SEQ ID NO: 2;
(b2) a protein having an amino acid sequence wherein 1 to 9 amino acids are deleted, substituted, inserted, and/or added in a region other than lysine at position 117 of the amino acid sequence represented by SEQ ID NO: 2; and
(b3) a protein having an amino acid sequence having at least 98% identity with the amino acid sequence represented by SEQ ID NO: 2, in which an amino acid at a position corresponding to position 117 of the amino acid sequence represented by SEQ ID NO: 2 is lysine.

4. The glycated protein according to claim 3,
wherein the protein according to any one of (b1) to (b3) is a barley-derived protein.

5. A rich flavor imparting agent comprising:
at least one of a glycated protein (A) or a glycated protein (B) below:
glycated protein (A): a glycated protein having a glycated side chain amino group of lysine, the glycated protein having a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
(a1) a protein having the amino acid sequence represented by SEQ ID NO: 1;
(a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
(a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine;
glycated protein (B): a glycated protein having a glycated side chain amino group of lysine, the glycated protein having a glycated side chain amino group of lysine at a position corresponding to position 117 of an amino acid sequence represented by SEQ ID NO: 2 of a protein according to any one of (b1) to (b3) below:
(b1) a protein having the amino acid sequence represented by SEQ ID NO: 2;
(b2) a protein having an amino acid sequence wherein 1 to 9 amino acids are deleted, substituted, inserted, and/or added in a region other than lysine at position 117 of the amino acid sequence represented by SEQ ID NO: 2; and
(b3) a protein having an amino acid sequence having at least 98% identity with the amino acid sequence represented by SEQ ID NO: 2, in which an amino acid at a position corresponding to position 117 of the amino acid sequence represented by SEQ ID NO: 2 is lysine.

6. The rich flavor imparting agent according to claim 5 for use in imparting a rich flavor to a beer-taste alcoholic beverage or a non-alcoholic beer-taste beverage.

7. Use of at least one of a glycated protein (A) or a glycated protein (B) below for imparting a rich flavor to a food or beverage:
glycated protein (A): a glycated protein having a glycated side chain amino group of lysine, the glycated protein having a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
(a1) a protein having the amino acid sequence represented by SEQ ID NO: 1;
(a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
(a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine;
glycated protein (B): a glycated protein having a glycated side chain amino group of lysine, the glycated protein having a glycated side chain amino group of lysine at a position corresponding to position 117 of an amino acid sequence represented by SEQ ID NO: 2 of a protein according to any one of (b1) to (b3) below:
(b1) a protein having the amino acid sequence represented by SEQ ID NO: 2;
(b2) a protein having an amino acid sequence wherein 1 to 9 amino acids are deleted, substituted, inserted, and/or added in a region other than lysine at position 117 of the amino acid sequence represented by SEQ ID NO: 2; and
(b3) a protein having an amino acid sequence having at least 98% identity with the amino acid sequence represented by SEQ ID NO: 2, in which an amino acid at a position corresponding to position 117 of the amino acid sequence represented by SEQ ID NO: 2 is lysine.

8. The use according to claim 7,
wherein the food or beverage is a beer-taste alcoholic beverage or a non-alcoholic beer-taste beverage.

9. A method of imparting a rich flavor to a food or beverage, the method comprising:
adding at least one of a glycated protein (A) or a glycated protein (B) below to a food or beverage:
glycated protein (A): a glycated protein having a glycated side chain amino group of lysine, the glycated protein having a glycated side chain amino group of at least one of lysine at a position corresponding to position 160 or lysine at a position corresponding to position 189 of an amino acid sequence represented by SEQ ID NO: 1 of a protein according to any one of (a1) to (a3) below:
(a1) a protein having the amino acid sequence represented by SEQ ID NO: 1;
(a2) a protein having an amino acid sequence wherein 1 to 9 amino acids are deleted, substituted, inserted, and/or added in a region other than at least one of lysine at position 160 or lysine at position 189 of the amino acid sequence represented by SEQ ID NO: 1; and
(a3) a protein having an amino acid sequence having at least 98% identity with the amino acid sequence represented by SEQ ID NO: 1, in which at least one of an amino acid at a position corresponding to position 160 or an amino acid at a position corresponding to position 189 of the amino acid sequence represented by SEQ ID NO: 1 is lysine;
glycated protein (B): a glycated protein having a glycated side chain amino group of lysine, the glycated protein having a glycated side chain amino group of lysine at a position corresponding to position 117 of an amino acid sequence represented by SEQ ID NO: 2 of a protein according to any one of (b1) to (b3) below:
(b1) a protein having the amino acid sequence represented by SEQ ID NO: 2;
(b2) a protein having an amino acid sequence wherein 1 to 9 amino acids are deleted, substituted, inserted, and/or added in a region other than lysine at position 117 of the amino acid sequence represented by SEQ ID NO: 2; and
(b3) a protein having an amino acid sequence having at least 98% identity with the amino acid sequence represented by SEQ ID NO: 2, in which an amino acid at a position corresponding to position 117 of the amino acid sequence represented by SEQ ID NO: 2 is lysine.

10. The method according to claim 9,
wherein the food or beverage is a beer-taste alcoholic beverage or a non-alcoholic beer-taste beverage.
